Europäisches Patentamt

(19) European Patent Office

Office européen des brevets

(11) Publication number : **0 200 360**
**B1**

## (12) EUROPEAN PATENT SPECIFICATION

(45) Date of publication of patent specification :
22.02.89

(51) Int. Cl.⁴ : **C 07 C 15/02**, C 07 C 7/13

(21) Application number : 86302285.1

(22) Date of filing : 26.03.86

(54) Process for the separation of C10 aromatic isomers.

(30) Priority : 26.03.85 US 716208

(43) Date of publication of application :
05.11.86 Bulletin 86/45

(45) Publication of the grant of the patent :
22.02.89 Bulletin 89/08

(84) Designated contracting states :
AT BE CH DE FR GB IT LI LU NL SE

(56) References cited :
FR–A– 2 147 201
FR–A– 2 148 567
FR–A– 2 304 383
US–A– 3 308 069
US–A– 4 554 398
(73) Proprietor : EXXON CHEMICAL PATENTS INC.
200 Park Avenue
Florham Park New Jersey 07932 (US)

(72) Inventor : Rosenfeld, Daniel David
16018 Mesa Verde Drive
Houston Texas (US)
Inventor : Daniel, Lawrence Gilbert
17523 Jolly Boat
Crosby Texas (US)

(74) Representative : Northover, Robert Frank et al
ESSO Chemical Limited Esso Chemical Research
Centre P.O. Box 1
Abingdon Oxfordshire, OX13 6BB (GB)

**Description**

This invention relates to hydrocarbon separations. More specifically, the claimed invention relates to the separation of durene from a hydrocarbon feedstream containing a mixture of $C_{10}$ aromatic isomers by use of the adsorbent zeolite beta which selectively removes durene from the feedstream. The selectively adsorbed durene is removed from the adsorbent through a desorption step. Durene is selectively removed from a mixture of $C_{10}$ aromatic isomers containing the tetramethylbenzene isomers. In general, paradiethylbenzene is preferentially adsorbed over the other $C_{10}$ aromatic isomers, with in one embodiment an order for decreased preference of adsorption on zeolite beta as follows : paradiethylbenzene > durene > metadiethylbenzene > orthodiethylbenzene and prehnitene > isodurene.

It is known in the separation art that certain adsorbents generally comprising crystalline aluminosilicates can be utilized to separate certain hydrocarbons from mixtures thereof. In the separation of aromatic hydrocarbon isomers with certain crystalline aluminosilicates containing selected cations at the zeolitic cationic sites, selectivity of the zeolite for a given aromatic isomer is enhanced. This manner of separation is particularly useful when the components to be separated have similar physical properties, such as freezing or boiling points, which renders the components difficult to separate by distillation or crystallization.

A number of processes describing the separation of paraxylene from a mixture of at least one other xylene isomer utilizing a crystalline aluminosilicate adsorbent, are shown in U.S. Patent Nos. 3,558, 730, 3,558,732, 3,626,020, and 3,663,638.

Other processes which describe the adsorption separation of ethylbenzene from a mixture of xylene isomers utilizing a crystalline aluminosilicate adsorbent are shown in U.S. Patent Nos. 3,943,182, 3,997,619, 3,998,901, and 4,021,499.

U.S. Patent No. 3,793,385 discloses the use of the crystalline aluminosilicate zeolite beta to separate $C_8$ aromatic isomers, specifically to separate paraxylene and ethylbenzene from a mixture containing at least one other $C_8$ aromatic isomer.

U.S. Patent No. 4,554,398 discloses a process for separating $C_9$ aromatic isomers from a feedstream containing a mixture thereof by contacting the feedstream with a bed of the adsorbent zeolite beta.

While the use of various zeolites to separate aromatic isomers is known, and the use of zeolite beta to separate $C_9$ aromatic isomers is known, less is known about adsorbents which effectively separate the $C_{10}$ aromatic isomers. FR-A-214201 describes a « para » selective separation process using zeolite X or Y and particularly the barium exchanged zeolite. It is described principally in relation to the separation of paraxylene, but is said to be effective at separating paradiethylbenzene from the ortho and meta isomers and butylbenzene.

The $C_{10}$ aromatic isomers are useful in the chemical arts as desorbents in separation processes and as precursors in preparing other chemicals. More specifically, durene is used in organic synthesis and in the manufacture of plasticizers, polymers and fibers, and isodurene is also used in organic synthesis. Paradiethylbenzene is useful as an intermediate or solvent. However, the availability of these $C_{10}$ aromatic isomers is restricted due to the difficulty of effectively separating a $C_{10}$ aromatic isomer from a mixture of the $C_{10}$ aromatic isomers.

Summary of the invention

It is accordingly a broad objective of this invention to provide a process of separating durene from a hydrocarbon feedstream containing a mixture of $C_{10}$ aromatic isomers.

In brief, the invention comprises and adsorptive separation process for the separation of durene and other $C_{10}$ aromatic isomers from a hydrocarbon feedstream containing a mixture of $C_{10}$ aromatic isomers by contacting the hydrocarbon feedstream with a bed of the adsorbent zeolite beta. A raffinate stream is then withdrawn from the bed, this stream containing less of the selectively adsorbed $C_{10}$ aromatic isomer. The adsorbed $C_{10}$ aromatic isomer on the bed is then desorbed to effect displacement of the isomer, followed by withdrawing from the adsorbent bed an extract stream containing the adsorbed $C_{10}$ aromatic isomer. The preferred zeolite beta adsorbent is cation exchanged to increase the $C_{10}$ aromatic selectivity of the adsorbent. the preferred $C_{10}$ aromatic isomer is durene. In general, paradiethylbenzene is preferentially adsorbed over the other $C_{10}$ aromatic isomers, with in one embodiment an order for decreased preference of adsorption on zeolite beta as follows : paradiethylbenzene > durene > metadiethylbenzene > orthodiethylbenzene and prehnitene > isodurene.

Detailed description of the invention

Hydrocarbon feedstreams which can be utilized in the process of this invention contain mixtures of $C_{10}$ aromatic isomers including durene. Specifically, these include the difficult-to-separate tetramethylbenzenes and dimethylethylbenzenes, and the diethylbenzenes. The tetramethylbenzene isomers include 1,2,4,5-tetramethylbenzene (durene), 1,2,3,5-tetramethylbenzene tetramethylbenzene (isodurene), and 1,2,3,4-tetramethylbenzene (prehnitene). The diethylbenzene isomers include 1,4-diethylbenzene (paradiethylbenzene), 1,3-diethylbenzene (metadiethylbenzene) and 1,2-diethylbenzene (orthodiethylbenzene). The close boiling points of these $C_{10}$ aromatic isomers (196 °C, 197 °C and 204 °C for the tetramethylbenzene isomers ; 181, 183, and 184 for the diethylbenzene isomers) render the isomers difficult to separate by distillation. Mixtures containing substantial quantities of $C_{10}$ aromatic isomers and other aromatics generally are produced by reforming processes, processes which are well known to the refining and petrochemical arts.

The hydrocarbon feedstream is contacted with a bed of the adsorbent, entitled zeolite beta. Zeolite beta and its method of manufacture are described in U.S. Patent 3,308,069 and its Reissue Patent 28,341 by Wadlinger et al., this disclosure being fully incorporated herein by reference. Also included with our definition of zeolite beta is where the aluminum is fully or partially substituted with the elements of gallium or boron to provide a gallosilicate or borosilicate, or where the silicon is fully or partially substituted with the elements of germanium, titanium or phosphorus but retains the same structure and similar X-ray diffraction pattern as defined in the Wadlinger et al. patents. After synthesizing the zeolite beta, it is necessary to calcine the zeolite at a temperature and for a time effective to remove any tetraethylammonium ions remaining after its synthesis. While the zeolite beta composition is fully described in this Wadlinger et al. patent, it has been surprisingly found that zeolite beta can be used to separate $C_{10}$ aromatic isomers from a feedstream containing a mixture of $C_{10}$ aromatic isomers. Further, the $C_{10}$ selectivity can be substantially increased by cation exchanging the zeolite beta with a suitable cation.

The process of this invention may be used to separate all the $C_{10}$ aromatic isomers from one another by the use of various stages or adsorption zones. When the $C_{10}$ aromatic isomers are tetramethylbenzenes they are selectively adsorbed in the order of durene and prehnitene > isodurene, with either durene or prehnitene being preferentially adsorbed depending upon the particular cation exchanged form of the zeolite beta and the conditions of adsorption. Preferably durene is selectively adsorption. Preferably durene is selectively adsorbed over prehnitene.

Diethylbenzenes are selectively adsorbed in the order of paradiethylbenzene > metadiethylbenzene and orthodiethylbenzene, with either metadiethylbenzene or orthodiethylbenzene being preferentially adsorbed depending on the particular cation exchanged form of zeolite beta and the conditions of adsorption. In general, paradiethylbenzene is preferentially adsorbed over the other $C_{10}$ aromatic isomers. In one embodiment, paradiethylbenzene is preferentially adsorbed over the other $C_{10}$ aromatic isomers with an order for decreased preference of adsorption on zeolite beta as follows : paradiethylbenzene > durene > metadiethylbenzene > orthodiethylbenzene and prehnitene > isodurene.

In order to substantially increase the selectivity of the adsorbent for $C_{10}$ aromatic isomers, the adsorbent which is available in its tetraethylammonium (TEA)-sodium form is preferably cation exchanged. After the TEA is removed the hydrogen-sodium form of zeolite beta can be exchanged with suitable cations which include base metal or transition metal cations, such as copper, rubidium, nickel, manganese, zinc, cobalt, potassium, cerium, barium, and cesium or mixtures thereof or other cations, such as ammonium. The preferred cations for increased selectivity of durene are hydrogen, sodium, cobalt and manganese with the most preferred cation being sodium. The preferred cation for increased selectivity of paradiethylbenzene is sodium. The zeolite beta can be exchanged with sodium by use of a sodium salt or by caustic treatment with sodium hydroxide.

The zeolite beta adsorbent can be combined with a binder, such as natural or synthetic clays (e. g., Kaolin), and inorganic oxides and can be in any form acceptable to the separation process such as extrudates, spheres, granules or tablets.

Certain characteristics of adsorbents are highly desirable, if not absolutely necessary, to the successful operation of a selective adsorption process. Among such characteristics are : adsorptive capacity for some weight of the $C_{10}$ aromatic isomer per weight of adsorbent ; and the selective adsorption of a $C_{10}$ aromatic isomer with respect to a raffinate component and the desorbent material.

Capacity of the adsorbent for adsorbing a specific volume of $C_{10}$ aromatic isomer is, of course, a necessity ; without such capacity the adsorbent is useless for adsorptive separation. Furthermore, the higher the adsorbent's capacity for the $C_{10}$ aromatic isomer, the better is the adsorbent. Increased capacity of a particular adsorbent makes it possible to reduce the amount of adsorbent needed to separate the $C_{10}$ aromatic isomer contained in a particular charge of feed mixture. A reduction in the amount of adsorbent required for a specific adsorptive separation reduces the cost of the separation process. It is important that the good initial capacity of the adsorbent be maintained during actual use in the separation process over some economically desirable life. Generally, the adsorbent of this invention has a capacity of at least 3 % of hydrocarbon by weight of adsorbent and preferably greater than 5 % of hydrocarbon by weight of adsorbent.

The second necessary adsorbent characteristic is the ability of the adsorbent to separate components of the feed ; or, in other words, that the adsorbent possess adsorptive selectivity, $(\alpha)$, for one component as compared to another component. Relative selectivity can be expressed not only for one feed component as compared to another but can also be expressed between any feed mixture component and the desorbent material. The separation factor, $(\alpha)$, as used throughout this specification is defined as the ratio of the two components of the adsorbed phase over the ratio of the same two components in the unadsorbed phase at equilibrium conditions.

Relative selectivity is shown as Equation 1 below :

$$\text{Selectivity} = (\alpha) = \frac{[\text{weight C/weight D}]_A}{[\text{weight C/weight D}]_U}$$

where C and D are two components of the feed represented by weight, and the subscripts A and U represent the adsorbed and unadsorbed phases, respectively. The equilibrium conditions were determined when the feed passing over a bed of adsorbent did not change composition after contacting the bed of adsorbent. In other words, there was no net transfer of material occurring between the unadsorbed and adsorbed phases.

Where selectivity of two components approaches 1.0 there is no preferential adsorption of one component by the adsorbent with respect to the other ; they are both adsorbed (or nonadsorbed) to about the same degree with respect to each other. As the selectivity ($\alpha$) becomes less than or greater than 1.0 there is preferential adsorption by the adsorbent for one component with respect to the other. When comparing the selectivity by the adsorbent of one component C over component D, an ($\alpha$) larger than 1.0 indicates preferential adsorption of component C within the adsorbent. An ($\alpha$) less than 1.0 would indicate that component D is preferentially adsorbed leaving an unadsorbed phase richer in component C and an adsorbed phase richer in component D. For optimum performance desorbent materials should have a selectivity equal to about 1 or less than 1 with respect to all extract components so that all of the extract components can be extracted as a class and all raffinate components clearly rejected into the raffinate stream. When the adsorbent of this invention is cation exchanged it is preferably exchanged with a cation which will impart an ($\alpha$) separation factor of at least 2.0 of the $C_{10}$ aromatic isomer (component C) over at least one of the other components (component D) of the hydrocarbon feedstream.

In order to test the various cation exchanged zeolite beta adsorbent materials with a particular feed mixture to measure the characteristics of adsorptive capacity and selectivity, a static testing procedure was employed. The procedure consisted of contacting a known weight of adsorbent with a known weight of mixed hydrocarbon feedstream. After allowing this mixture to reach equilibrium, a sample was removed and analyzed by gas chromatography. The amount of isomers in the raffinate were measured and the amount of isomers adsorbed were obtained by difference from the standard feedstream.

In a separation process, after the hydrocarbon feedstream is contacted with the bed of adsorbent, a raffinate stream is withdrawn from the adsorbent bed, this stream containing less of the selectively adsorbed $C_{10}$ aromatic isomer of the feedstream. then the adsorbed aromatic isomer on the bed is desorbed to effect displacement thereof.

The desorbing step which can be used in the various processing schemes employing this adsorbent will vary depending on the type of operation employed. the term « desorbent material » as used herein shall mean any fluid substance capable of removing a selectively adsorbed $C_{10}$ aromatic isomer from the adsorbent. In the swingbed system in which the selectively adsorbed $C_{10}$ aromatic isomer is removed from the adsorbent by a purge stream, desorbent materials comprising gaseous hydrocarbons such as methane, ethane, or other types of gases such as nitrogen or hydrogen may be used at elevated temperatures or reduced pressures or both to effectively purge the adsorbed $C_{10}$ aromatic isomer from the adsorbent.

However, in an adsorptive separation process which employs the adsorbent and which is generally operated at substantially constant pressures and temperatures to insure a liquid phase, the desorbent material relied upon must be judiciously selected to satisfy several criteria. First, the desorbent material must displace the $C_{10}$ aromatic isomer from the adsorbent with reasonable mass flow rates without itself being so strongly adsorbed as to unduly prevent the $C_{10}$ aromatic isomer from displacing the desorbent material in a following adsorption cycle. Expressed in terms of the selectivity, it is preferred that the adsorbent be more selective for a $C_{10}$ aromatic isomer with respect to a raffinate (e. g., other isomers), than it is for the desorbent material with respect to a raffinate. Secondly, desorbent materials must be compatible with the particular adsorbent and the particular feedstream. More specifically they must not reduce or destroy the critical selectivity of the adsorbent for the $C_{10}$ aromatic isomer with respect to the raffinate.

Desorbent materials to be used in the process of this invention should additionally be substances which are easily separable from the feedstream that is passed into the process. After desorbing the $C_{10}$ aromatic isomer of the feed, both desorbent material and the $C_{10}$ aromatic isomers are removed in a mixture from the adsorbent. Without a method of separating the desorbent material, such as distillation, the purity of either the $C_{10}$ aromatic isomer or the raffinate component would not be very high. it is therefore contemplated that any desorbent material used in this process will have a substantially different average boiling point than that of the feedstream. The use of a desorbent material having a substantially different average boiling point than that of the feed allows separation of desorbent material from feed components in the extract and raffinate streams by simple fractionation, thereby permitting reuse of desorbent material in the process. The term « substantially different » as used herein shall mean that the difference between the average boiling points between the desorbent material and the feed mixture shall be at least 15 °F (— 9.5°). The boiling range of the desorbent material may be higher or lower than that of the feed mixture.

EP 0 200 360 B1

In a liquid-phase operation of the process of our invention, desorbent materials comprising monoaromatic hydrocarbons are effective. Mixtures of the aromatic hydrocarbon with paraffins are also effective as desorbent materials. Such paraffins must be compatible with the adsorbent and feedstream as described above and must be easily separated from the feedstream. The paraffins can include straight or branched chain paraffins (such as heptane) or cycloparaffins which meet these criteria. Typical concentrations of aromatics in such mixtures can be from a few volume percent up to 100 volume % of the total desorbent material mixture but such concentrations preferably will be within the range of from 50 volume % to 100 volume % of the mixture. The desorbents may be benzene, alkylbenzene, polyalkylbenzenes such as diethylbenzene, and dimethylbenzenes or xylenes, or more generally polycyclic hydrocarbons and mixtures thereof. The suitable desorbents include toluene and ethyltoluenes, with metaxylene or orthoxylene preferred as they provide increased separation and resolution of separation of the $C_{10}$ aromatic isomers.

Following desorption, the extract stream containing the $C_{10}$ aromatic isomer is withdrawn from the adsorbent bed. Depending on the separation factor ($\alpha$) this withdrawn extract can contain relatively pure fractions of $C_{10}$ aromatic isomer. However, it will be appreciated that the selectively adsorbed component is generally not completely adsorbed by the adsorbent, nor is the raffinate component generally completely non-adsorbed by the adsorbent.

In general, this adsorptive separation process can be carried out in the vapor or liquid phase, while the liquid phase is preferable. Adsorptive conditions for the process of this invention may include temperatures within the range of from ambient to 300 °C and will include pressures in the range from about atmospheric to 3447.5 kPa (500 psig).

In a preferred embodiment the process is carried out in the liquid phase at temperatures of 90-200 °C to lower the viscosity of the feedstream allowing for an increased flow rate or a reduction in the diameter of the adsorption column. Desorption conditions for the process of this invention shall generaly include the same range of temperatures and pressures as described for the adsorption operation. The desorption of the selectively adsorbed $C_{10}$ aromatic isomer could also be effected at subatmospheric pressures or elevated temperatures or both, or by vacuum purging of the adsorbent to remove the adsorbed isomer, but this process is not primarily directed to these desorption methods.

Example I

A crystalline aluminosilicate zeolite beta adsorbent in its hydrogen form was cation exchanged with the cations as listed in Table I (the NaOH in parentheses indicates Na exchange was carried out with NaOH instead of a sodium salt). A $C_{10}$ aromatic feedstream containing 1 % of durene, 2 % of isodurene, 2 % of prehnitene, and 0.5 % of triisopropyl benzene (an internal standard for the gas chromatograph) with the remainder heptane, all by weight, was added at ambient temperature to the various cation exchanged zeolite beta adsorbents, with the amount of feedstream being in excess of that which the zeolite can adsorb. After allowing this mixture to reach equilibrium, the mixture was allowed to settle and a sample was removed and analysed by gas chromatography. The amount of $C_{10}$ isomers in the raffinate was measured and the amount of isomers adsorbed was obtained by difference from the standard feedstream. The capacity and the ($\alpha$) separation factor were calculated for the tetramethylbenzene isomers as listed in Table I.

Table I

($\alpha$) Separation Factor

| Cation Form of Zeolite Beta | Durene/ Isodurene | Durene/ Prehnitene | Prehnitene/ Isodurene | Adsorption Capacity, wt% |
|---|---|---|---|---|
| H | 5.9 | 3.3 | 1.8 | 6 |
| Na (NaOH) | 15.5 | 5.3 | 2.9 | 8 |
| Na | 9.1 | 3.9 | 2.4 | 7 |
| Co | 4.5 | 4.4 | 1.0 | 3 |
| Mn | 10.1 | 2.0 | 4.9 | 3 |
| Cs | 1.3 | 0.6 | 2.1 | 5 |
| Ce | 1.4 | 0.7 | 2.1 | 4 |
| Rb | 1.1 | 0.7 | 1.5 | 7 |
| Ni | 2.2 | 0.9 | 2.5 | 6 |
| Zn | 3.5 | 1.0 | 3.4 | 4 |
| Cu | 2.2 | 0.6 | 3.2 | |
| Ba | 6.9 | 0.8 | 8.6 | 4 |

5

As can be seen from Table I, the sodium exchanged form of zeolite beta was highly effective in the separation of durene from the other tetramethylbenzene isomers.

Example II

In two runs a sample of a $C_{10}$ aromatic feed (b. p. 192-202 °C) was obtained from a plant which contained 44.1 wt% of the three isomeric tetramethylbenzenes, 18.4 wt% of the dimethylethylbenzenes, 1.4 wt% naphthalene as well as 36.1 wt% of five other unidentifiable components (components 1-5). The feed was added to a sodium exchanged aluminosilicate zeolite beta adsorbent as in Example I. After allowing the mixture to reach equilibrium a sample was removed and analyzed by gas chromatography. The ($\alpha$) separation factors of durene to the other components of the feed are listed on the following Table II.

Table II

($\alpha$). Separation Factor

|  | Run 1 | Run 2 |
|---|---|---|
| Durene/Isodurene | 8.7 | 9.8 |
| Durene/Prehnitene | 3.8 | 3.6 |
| Durene/Component 1 | 4.4 | 4.4 |
| Durene/Component 2 | 19.1 | 22.8 |
| Durene/Component 3 | 24.2 | 22.4 |
| Durene/Component 4 | 4.2 | 4.1 |
| Durene/Component 5 | 1.6 | 1.8 |
| Durene/(1,4-DM-2-EB) | 2.5 | 2.3 |
| Durene/(1,3-DM-4-EB) | 3.2 | 3.4 |
| Durene/(1,2-DM-3-EB) | 8.4 | 10.2 |
| Durene/(1,2-DM-4-EB) | 3.6 | 3.1 |
| Durene/(naphthalene) | 3.6 | - |

This experiment shows the sodium zeolite beta adsorbent is more selective to durene than to any of the other components of the plant feed.

Based on the separation factor of durene relative to the dimethylethylbenzene isomers it is apparent that sodium zeolite beta is selective to the dimethylethylbenzene isomers in the following oder of decreasing preference of adsorption on zeolite beta : durene > 1,4-dimethyl-2-ethylbenzene > 1,3-dimethyl-4-ethylbenzene and 1,2-dimethyl-1,4-ethylbenzene > 1,2-dimethyl-3-ethylbenzene.

Example III

A crystalline gallosilicate zeolite beta adsorbent in its hydrogen form was cation exchanged with the cations as listed in Table III. A $C_{10}$ aromatic feedstream as in Example I was added and analyzed as in Example I. The ($\alpha$) separation factor was calculated for the tetramethylbenzene isomers as listed in Table III.

Table III

($\alpha$) Separation Factor

| Cation Form of Zeolite Beta | Durene/ Isodurene | Durene/ Prehnitene | Prehnitene/ Isodurene |
|---|---|---|---|
| H | 7.6 | 2.0 | 3.7 |
| Co | 3.5 | 1.4 | 2.5 |
| K | 1.4 | 0.9 | 1.6 |
| Na (NaOH) | 4.1 | 2.7 | 1.5 |

This experiment shows the gallosilicate zeolite beta adsorbent is effective in separating the tetramethylbenzene isomers.

## Example IV

A sample of a $C_{10}$ aromatic feedstream containing equimolar amounts of durene, isodurene, prehnitene and metaxylene was added to a sodium exchanged aluminosilicate zeolite beta adsorbent at various temperatures in an amount equal to the capacity of the adsorbent. After agitation to reach equilibrium a gas phase sample was removed and analyzed by gas chromatography. From the peaks of the chromatograms the ($\alpha$) separation factors between the various components of the feedstream were measured. The capacity ranged from 18 to 22 wt%. The ($\alpha$) separation factor is listed on the following Table IV :

### Table IV

#### ($\alpha$) Separation Factor

| Temperature | Durene/ Isodurene | Durene/ Prehnitene | Durene/ Metaxylene |
|---|---|---|---|
| 60°C | 11 | 6.8 | 7.5 |
| 80°C | 9.2 | 6.5 | 5.3 |
| 140°C | 6 | 5.1 | 2.5 |

## Example V

A three foot section of liquid chromatography tubing was packed with crystals of a sodium exchanged aluminosilicate zeolite beta adsorbent. A plant $C_{10}$ aromatic feed as in Example II was run through the column at 90 ºC and was desorbed with a desorbent containing 100 % metaxylene. The ($\alpha$) separation factor for durene/isodurene was 6.1 and for durene/prehnitene was 4.5. Durene was recovered at a purity of greater than 95 % by weight of the $C_{10}$ aromatic feed.

## Example VI

A three foot section of liquid chromatography tubing was packed with particles of sodium zeolite beta. A feed comprising equal weights of triisopropylbenzene, paradiethylbenzene, metadiethylbenzene, orthodiethylbenzene, durene, isodurene and prehnitene, was run through the column at 90 ºC and was desorbed with 100 % orthoxylene. 100 % of the paradiethylbenzene present in the feedstream was recovered at a purity of 100 %. 97 % of the durene present in the feedstream was recovered as a separate product at a purity of 100 %. The ($\alpha$) separation factors of the components were as follows :

### Table IX

#### ($\alpha$) Separation Factor

| Component | P-diethylbenzene/ Component | Durene/ Component |
|---|---|---|
| Isodurene | 64 | 22 |
| Prehnitene | 21 | 7 |
| O-diethylbenzene | 21 | 7 |
| M-diethylbenzene | 13 | 4 |
| Durene | 3 | — |
| P-diethylbenzene | — | 0.3 |

## Claims

1. An adsorptive separation process for separating at least durene from a hydrocarbon feedstream containing a mixture of $C_{10}$ aromatic isomers comprising :
   (a) contacting said hydrocarbon feedstream with a bed of an adsorbent of zeolite beta ;
   (b) withdrawing from said bed of adsorbent a raffinate stream containing less of the selectively adsorbed durene of the feedstream ;
   (c) desorbing the adsorbed durene to effect displacement thereof ; and
   (d) withdrawing from the adsorbent bed an extract stream containing the adsorbed durene.

2. Process of claim 1 wherein the mixture of $C_{10}$ aromatic isomers contains tetramethylbenzenes which are selectively adsorbed in the order of durene and prehnitene > isodurene.

3. Process of either of claims 1 and 2 in which said adsorbent contains at least one cation selected from the group consisting of hydrogen, the Group 1 and Group II metals, and the transition metals.

4. Process of any of claims 1 to 3, wherein the adsorbed aromatic components are desorbed by passing a desorbent material through said bed.

5. Process of claim 4 wherein the desorbent is selected from the group consisting of toluene, benzene, dimethylbenzene, paraffin, ethyltoluene, diethylbenzene, alkylbenzenes, polyalkylbenzenes, polycyclic hydrocarbons and mixtures thereof.

6. Process of any of claims 1 to 5 wherein the separation process is carried out at a temperature within the range of ambient to 300 °C and a pressure within the range of atmospheric to 3447.5 kPa (500 psig).

7. Process of claim 6 wherein the process is carried out at a temperature within a range of 90 to 200 °C.

8. Process of any of claims 1 to 7 wherein the adsorbent is combined with a binder.

9. Process of any of claims 1 to 8 in which said adsorbent has a capacity of at least 3 % of hydrocarbon by weight of adsorbent.

10. Process of claim 3, wherein the zeolite beta has been cation exchanged by use of a sodium hydroxide or a sodium salt.

**Patentansprüche**

1. Adsorptionstrennungsverfahren zur Abtrennung von wenigstens Durol aus einem Kohlenwasserstoffbeschickungsstrom, enthaltend eine Mischung von aromatischen $C_{10}$-Isomeren, umfassend :

(a) Den Kohlenwasserstoffbeschickungsstrom mit einem Bett des Adsorptionsmittels aus Zeolith beta in Kontakt bringen.

(b) Von diesem Adsorptionsmittelbett ein Raffinatstrom abziehen, der weniger des selektiv adsorbierten Durols aus dem Beschickungsstrom enthält,

(c) das adsorbierte Durol desorbieren, um seine Verdrängung davon zu bewirken und

(d) vom Adsorptionsbett einen Extraktstrom abziehen, enthaltend das adsorbierte Durol.

2. Verfahren gemäß Anspruch 1, worin die Mischung aus aromatischen $C_{10}$-Isomeren Tetramethylbenzole enthält, welche selektiv gemäß der Reihe Durol und Prehnitol > Isodurol adsorbiert werden.

3. Verfahren gemäß einem der Ansprüche 1 und 2, worin das Adsorptionsmittel wenigstens 1 Kation, ausgewählt aus der Gruppe, bestehend aus Wasserstoff, der Gruppe 1 und der Gruppe 2 der Metalle und der Übergangstalle, enthält.

4. Verfahren gemäß einem der Ansprüche 1 bis 3, worin die adsorbierten aromatischen Komponenten desorbiert werden, indem ein Desorptionsmaterial durch das Bett geleitet wird.

5. Verfahren gemäß Anspruch 4, worin das Desorptionsmittel ausgewählt wird aus der Gruppe, bestehend aus Toluol, Benzol, Dimethylbenzol, Paraffin, Ethyltoluol, Diethylbenzol, Alkylbenzole, Polyalkylbenzole, polycyklische Kohlenwasserstoffe und deren Mischungen.

6. Verfahren gemäß einem der Ansprüche 1 bis 5, worin das Trennungsverfahren bei einer Temperatur im Bereich von Raumtemperatur bis 300 °C und einem Druck im Bereich von Atmosphärendruck bis 3447,5 kPa (500 psig) durchgeführt wird.

7. Verfahren gemäß Anspruch 6, worin das Verfahren bei einer Temperatur im Bereich von 90 bis 200 °C durchgeführt wird.

8. Verfahren gemäß einem der Ansprüche 1 bis 7, worin das Adsorptionsmittel mit einem Bindemittel kombiniert wird.

9. Verfahren gemäß einem der Ansprüche 1 bis 8, worin das Adsorptionsmittel eine Kapazität von wenigstens 3 % der Kohlenwasserstoffe bezogen auf Gew.-% Adsorptionsmittel hat.

10. Verfahren gemäß Anspruch 3, worin das Zeolith-β kationenausgetauscht worden ist durch Verwendung von Natriumhydroxid oder eines Natriumsalzes.

**Revendications**

1. Procédé de séparation par adsorption, destiné à séparer au moins le durène d'une charge hydrocarbonée contenant un mélange d'isomères aromatiques en $C_{10}$, qui consiste :

(a) à faire entrer ladite charge hydrocarbonée en contact avec un lit d'une matière adsorbante formée de zéolite bêta ;

(b) à décharger du lit de matière adsorbante un courant de raffinat contenant une moins grande quantité du durène sélectivement adsorbé du courant de charge ;

(c) à désorber la durène adsorbé pour effectuer son déplacement ; et

(d) à décharger du lit de matière adsorbante un courant d'extrait contenant le durène adsorbé.

2. Procédé suivant la revendication 1, dans lequel le mélange d'isomères aromatiques en $C_{10}$

contient des tétraméthylbenzènes qui sont sélectivement adsorbés dans l'ordre durène et prehnitène > isodurène.

3. Procédé suivant l'une des revendications 1 et 2, dans lequel ladite matière adsorbante contient au moins un cation choisi dans le groupe comprenant l'hydrogène, les métaux des Groupes I et II et les métaux de transition.

4. Procédé suivant l'une quelconque des revendications 1 à 3, dans lequel les composants aromatiques adsorbés sont désorbés par passage d'une matière désorbante à travers le lit.

5. Procédé suivant la revendication 4, dans lequel la matière désorbante est choisie dans le groupe comprenant le toluène, le benzène, le diméthylbenzène, une paraffine, l'éthyltoluène, le diéthylbenzène, des alkylbenzènes, des polyalkylbenzènes, des hydrocarbures polycycliques et leurs mélanges.

6. Procédé suivant l'une quelconque des revendications 1 à 5, dans lequel la séparation est effectuée à une température comprise dans un intervalle de la température ambiante à 300 °C et à une pression allant de la pression atmosphérique à une pression manométrique de 3 447,5 kPa (500 lb/in$^2$).

7. Procédé suivant la revendication 6, dans lequel l'opération est mise en œuvre à une température comprise dans un intervalle de 90 à 200 °C.

8. Procédé suivant l'une quelconque des revendications 1 à 7, dans lequel la matière adsorbante est associée à un liant.

9. Procédé suivant l'une quelconque des revendications 1 à 8, dans lequel la matière adsorbante a une capacité, par rapport à son poids, d'au moins 3 % d'hydrocarbure.

10. Procédé suivant la revendication 3, dans lequel la zéolite bêta a été traitée par échange cationique par l'utilisation d'un hydroxyde de sodium ou d'un sel de sodium.